# EUROPÄISCHE PATENTSCHRIFT

(11) **EP 2 152 325 B1**
(45) Veröffentlichungstag und Bekanntmachung des Hinweises auf die Patenterteilung: **09.04.2014**
(21) Anmeldenummer: 08735371.0
(22) Anmeldetag: 23.04.2008
(51) Int. Cl.: A61L 2/24, B65B 55/10

(54) **VERFAHREN ZUM STERILISIEREN VON BEHÄLTERN**
CONTAINER STERILISATION METHOD
PROCEDE DE STERILISATION DE CONTENANTS

(30) Priorität: 27.04.2007 DE 102007020457
(43) Veröffentlichungstag der Anmeldung: 17.02.2010
(73) Patentinhaber: KHS GmbH, 44143 Dortmund (DE)
(72) Erfinder: SANGI, Daryoush, 20251 Hamburg (DE); HEROLD, Thomas, 22926 Ahrensburg (DE)
(86) Internationale Anmeldenummer: PCT/EP2008/003247
(87) Internationale Veröffentlichungsnummer: WO 2008/135165

(56) Entgegenhaltungen:
- EP-A- 0 590 505
- DE-A1- 19 949 692
- DE-A1-102004 030 956
- DE-A1-102004 030 957
- US-A1- 2006 032 189

## Beschreibung

Die Erfindung bezieht sich auf ein Verfahren zum Sterilisieren von Behältern gemäß Oberbegriff Patentanspruch 1 sowie auf eine Sterilisierungsvorrichtung gemäß Oberbegriff Patentanspruch 14.

Verfahren zum Sterilisieren von Flaschen, Dosen oder dergleichen Behältern unter Verwendung eines Wasserstoffperoxyd (H2O2) enthaltenden Sterilisationsmedium, d.h. unter Verwendung eines Sterilisationsmediums (nachstehend auch H2O2-Sterilisatinsmedium), welches Wasserstoffperoxyd in Mischung mit heiße steriler Luft enthält, sind bekannt (DE 10 2004 030 958 A1, DE 10 2004 030 957 A1, DE 199 49 692 A1, WO 2006/053745 A1). Bei diesen Verfahren, die z.B. zum Sterilisieren von Behältern für Getränke, aber auch zum Sterilisieren von Behältern oder Verpackungen für andere Produkte, wie z.B. Arzneimittel, verwendet werden, wird beim Einbringen des heißen H2O2-Sterilisatinsmedium an der Innenfläche des kühleren Behälters durch Kondensation ein H2O2-Kondensationsfilm gebildet, der dann in einer darauf folgenden Aktivierungsphase durch Einbringen eines sterilen heißen gas- und/oder dampfförmigen Aktivierungsmediums, beispielsweise durch Einbringen von heißer steriler Luft in der Weise aktiviert wird, dass durch Zerfall von H2O2 freie Sauerstoffradikale entstehen, die zur Sterilisation der Behälter mit vorhandenen Keimen und Verunreinigungen reagieren.

Bekannt ist bel diesen Verfahren auch (DE 10 2004 030 956 A1), die als Aktivierungsmedium verwendete sterile Luft auf Aktivierungstemperatur dadurch zu erhitzen, dass sie durch einen auf eine Temperatur zwischen 130°C und 150°C erhitzten Wärmetauscher geleitet wird. In einem auf die Aktivierungsphase folgenden Verfahrensschritt erfolgt dann ein Ausblasen und Abkühlen der Behälter mit der sterilen Luft, die den Behälter mit einer Temperatur deutlich unter der Aktivierungstemperatur zugeführt wird. Die sterile Luft wird hierfür mit einem entsprechend hohen Volumenstrom, der ein Erhitzen auf die Aktivierungstemperatur verhindert, durch den Wärmetauscher geleitet.

Bekannt sind weiterhin Verfahren und Vorrichtungen (EP 0 590 505 A1, DE 198 46 322 A1) zur Behandlung von Flaschen oder dergleichen Behältern mit einem heißen Behandlungsmedium, welches in die Behälter eingebracht wird. Mit Hilfe von Temperatursensoren wird die Temperatur der Behälter vor und nach der Wärmebehandlung bzw. die Temperatur des rückfließenden Behandlungsmediums erfasst und u.a. die Temperatur des heißen Behandlungsmediums und/oder die Intensität der Behandlung In Abhänglgkeit von den gemessenen Temperaturen gesteuert. Eine Behandlung mit einem Wasserstoffperoxyd enthaltenden Behandlungsmedium ist nicht vorgesehen.

Aufgabe der Erfindung ist es, ein Verfahren und eine Vorrichtung aufzuzeigen, womit unter Aufrechterhaltung einer hohen Entkeimungsrate, d.h. einer hohen Qualität der Sterilisation die Behandlungsdauer insgesamt und dabei speziell auch die Dauer der Aktivierungsphase reduziert werden kann, und zwar bei schonender Behandlung der Behälter. Zur Lösung dieser Aufgabe ist ein Verfahren entsprechend dem Patentanspruch 1 ausgebildet. Die Vorrichtung ist gemäß dem Anspruch 14 ausgebildet, wobei die nachfolgenden abhängigen Ansprüche Ausführungsvarianten aufzeigen.

Mit dem erfindungsgemäßen Verfahren und der Vorrichtung ergibt sich u.a. eine erhebliche Reduzierung der Verfahrensdauer und dabei speziell der Gesamtdauer der Aktivierungsphase. Gleichzeitig erfolgt auch eine schonende und eine thermische Überlastung der Behälter vermeidende Behandlung bei hoher Qualität der Sterilisation bzw. bei hoher Entkeimungsrate. Das erfindungsgemäße Verfahren eignet sich daher insbesondere für Behälter aus Kunststoff, z.B. aus PET.

Weiterbildungen der Erfindung sind Gegenstand der Unteranspruche. Die Erfindung wird im Folgenden im Zusammenhang mit den Figuren an einem Ausführungsbeispiel näher erläutert, Es zeigen:
- Fig. 1: in sehr vereinfachter Darstellung und in Draufsicht eine Maschine bzw. Vorrichtung zum Durchführen des erfindungsgemäßen Verfahrens;
- Fig. 2: in vereinfachter Darstellung einen Behandlungskopf der Vorrichtung der Figur 1.

Die in den Figuren allgemein mit 1 bezeichnete Vorrichtung zur Sterilisierung von Flaschen bzw. das Verfahren zeigt einen um eine vertikale Maschinenachse umlaufend antreibbaren Rotor 2 zur Applikation des Sterilisierungsmittels in die zu behandelnden Flaschen 3, die über einen Behältereinlaufstern 4 zugeführt und aus dem die behandelten, d.h. die benetzten Flaschen 3 über einen Behälterauslauf 5 entnommen und dem nachfolgenden Aktivator zugeführt werden. Der Aktivator ist ebenfalls ein um die vertikale Maschinenachse umlaufend antreibbarer Rotor 6 zur Aktivierung des Sterilisierungsmittels mittels steriler Heizluft, die in die zu behandelnden Flaschen 3 geleitet wird. Die Flaschen 3 werden über einen Behältereinlaufstern 7 dem Rotor 6 zugeführt und die behandelten, d.h. sterilisierten Flaschen 3 über einen Behälterauslauf 8 entnommen und dem nachfolgenden Verfahrensstufe, in der Regel einem Füller, zugeführt.

Oberhalb jeder Flaschenöffnung sind in bekannter Weise am Rotor 2 Applikationsköpfe vorgesehen, welche mit dem Rotor 2 umlaufen und nur als doppelte, gestrichelte Linien I angedeutet sind. Jedem Applikationskopf ist am Rotor 2 ein Flaschen- oder Behälterträger 14 zugeordnet, an dem die jeweilige Flasche 3 während der Behandlung unterhalb des Behandlungskopfes 6 gehalten wird und zwar bei der beispielhaften Ausführungsform, eine als PET-Flaschen ausgebildeten Flaschen 3, an einem flaschenseitigen Mündungsflansch hängend.

Die Benetzung der Oberflächen der Flaschen 3 erfolgt unter Verwendung des H₂O₂-Sterilisationsmedium, welches in bekannter Weise innerhalb des jeweiligen Behandlungskopfes durch Einsprühen von Wasserstoffperoxyd, beispielsweise von 35%-igem Wasserstoffperoxyd in sterile Luft und durch Erhitzen des so erhaltenen Aerosols auf eine Temperatur T₁ von beispielsweise 145°C erwärmt wird.

Für die Behandlung wird heißes H₂O₂-Sterilisationsmedium in das Innere der Flasche 3 eingebracht, und zwar derart, dass sich durch Kondensation an der im Vergleich zur Temperatur T₁ des H₂O₂-Sterilisationsmediums kälteren Innenfläche der Flasche 3 ein H₂O₂-Kondensationsfilm bildet, der zumindest die gesamte Innenfläche der jeweiligen Flasche 3 mit einem H₂O₂-Kondensationsfilm gleichmäßig überzieht.

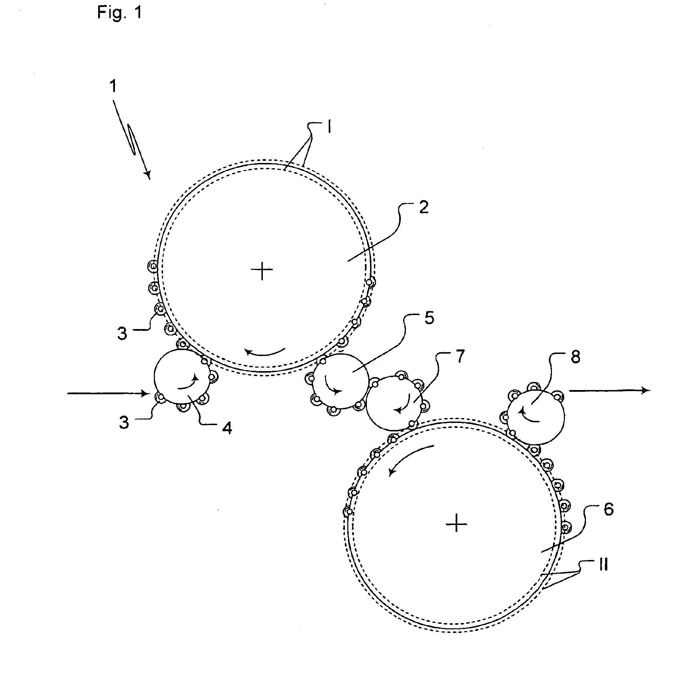

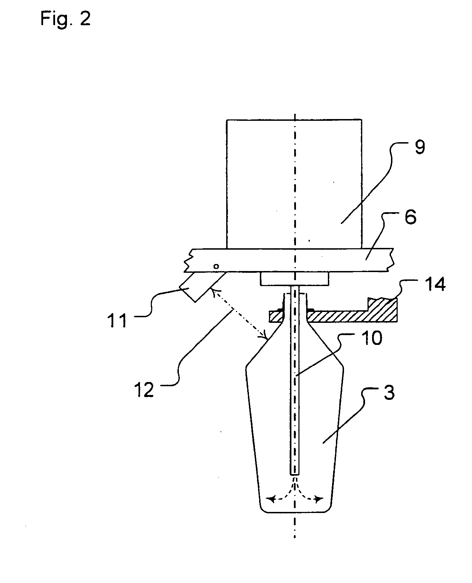

Im Anschluss an diese Applikationsphase und nach der Überleitung der so benetzten Flaschen auf den Rotor 6 erfolgt dann in einer weiteren Behandlungsphase, d.h. in einer Aktivierungsphase, eine Aktivierung des H₂O₂-Kondensationsfilmes. Hierzu sind analog dem Aufbau und Anordnung am Rotor 2 oberhalb jeder Flaschenöffnung in bekannter Weise Aktivatorköpfe 9 vorgesehen, welche mit dem Rotor 6 umlaufen und nur in Fig. 1 als doppelte, gestrichelte Linien II angedeutet sind. Die Aktivierung wird durch Energieeintrag gestartet und zwar durch Einleitung eines heißen sterilen gas- und/oder dampfförmigen Mediums, beispielsweise durch Einleitung von heißer steriler Luft mit einer Temperatur T₂ in die jeweilige Flasche 3 und zwar durch ein in diese Flasche eingeführtes Rohr 10 (Fig. 2). Mit dieser Aktivierung erfolgt eine Zerfallreaktion von H₂O₂, in deren Verlauf u.a. freie Sauerstoff-Radikale entstehen, die mit vorhandenen Keimen und/oder Verunreinigungen in der jeweiligen Falsche 3 reagieren und so deren Sterilisation bewirken. Mit der in der Aktivierungsphase verwendeten heißen sterilen Luft erfolgt zugleich auch ein Trocknen der jeweiligen Flasche 3. Sterilisationsverfahren mit diesen Verfahrensschritten sind grundsätzlich bekannt.

Das erfindungsgemäße Verfahren zeichnet sich gegenüber bekannten Verfahren durch eine spezielle Ausgestaltung der einzelnen Behandlungsphasen bzw. deren Verfahrensschritte aus.

In der Applikationsphase erfolgt das Einbringen des heißen H₂O₂-Sterilisationsmediums mit einer konstanten Temperatur T₁ und mit einer konstanten Abgabe- oder Applikationszeit, beispielsweise mit einer Abgabezeit von 3sec bei Flaschen oder Behältern mit einem Volumen von 500 ml. Der Volumenstrom v₁ des in die jeweilige Falsche 3 eingebrachten heißen H₂O₂-Sterilisationsmediums ist dabei beispielsweise während der Applikationszeit ebenfalls konstant.

Das Aktivieren des H₂O₂-Kondensationsfilmes in der jeweiligen Flasche 3 erfolgt während der Aktivierungsphase in zwei Verfahrens-/Aktivierungsschritten. Während eines ersten Verfahrensschrittes wird die zur Aktivierung verwendete heiße sterile Luft mit einer Temperatur T₂ mit einem konstanten, großen Volumenstrom v₂ über das Rohr 10 (Fig. 2) in die Flasche 3 eingebracht. Dieses Einbringen erfolgt beispielsweise über eine vorgegebene Abgabezeit von x Sekunden. oder aber solange, bis die Temperatur der Behälterwandung T_{BW} der jeweiligen Flasche 3 eine vorgegebene Solltemperatur Soll-T_{BW} erreicht hat, die über einen Pyrometer 11 (Fig. 2) gemessen bzw. überwacht wird. Der strichpunktierte Pfeil 12 skizziert den Messvorgang. Die Gesamtdauer dieses ersten Verfahrensschrittes ist ca. 8 bis 10 Sekunden.

In einem anschließenden weiteren Verfahrensschritt wird dann das heiße Aktivierungsmedium, welches bevorzugt wiederum heiße sterile Luft ist, mit der Temperatur T₃ und mit einem Volumenstrom v₃ in die Flasche 3 eingebracht und zwar über eine Abgabezeit von y Sekunden. Die Temperatur des Volumenstroms v₃, der in der Regel gleich oder auch kleiner ist als der Volumenstrom v₂, wird dabei in Abhängigkeit von der Behältertemperatur T_{BW} der jeweiligen Flasche 3 abgesenkt, so dass auch während dieses zweiten Verfahrensschrittes der Aktivierungsphase die Behältertemperatur T_{BW} die Solltemperatur Soll-T_{BW} aufweist bzw. unter einem maximal zulässigen Wert bleibt. T₃ ist somit kleiner als T₂ und wird über einen Wärmetauscher und/oder die Beimischung von kalter Sterilluft oder einem kalter Inertgas, wie CO₂ oder N₂ eingestellt.

Die Solltemperatur Soll-T_{BW} liegt dabei in beiden Verfahrensschritten unter einer Temperatur, die zu einer übermäßigen Belastung oder Verformung oder zu einer Beschädigung der Flaschen 3 führen würde.

Die Behältertemperatur T_{BW} wird auch im zweiten Verfahrensschritt berührungslos unter Verwendung wenigstens eines Pyrometers 11 gemessen, wie dies in der Figur 2 angedeutet ist Abhängig von der Konstruktion des Rotors 6 und dem dazu gehörigen Aktivatorkopf sowie dem Behältermaterial, können auch andere berührungslose Wärmemesssysteme verwendet werden. Bei der in Fig. 2 gezeigten Variante ist der Pyrometer 11 schwenkbar gelagert, um abhängig von der Flaschen- oder Behältergeometrie optimal ausgerichtet werden zu können.

Da die Temperatur der Flaschenwandung überwacht wird, kann ein derart steiler Erwärmungsgradient und Temperatur in der zweiten Aktivierungsphase gewählt werden, was in bekannten Anlagen aus Sicherheitsgründen (Verformung der Flasche) nicht einstellbar war.

Die Vorteile des erfindungsgemäßen Verfahrens bestehen u.a. darin, dass die Aktivierung viel heftiger abläuft und auf sehr hohem Niveau gehalten werden kann, so dass eine wesentliche Verkürzung der Behandlungsdauer möglich ist, d.h. eine Verkürzung unter 10 s ergibt sich. Insbesondere im größeren Leistungsbereich, d.h. bei höherer Leistung der die Vorrichtung 1 aufweisenden Anlage (Anzahl der verarbeiteten Flaschen 3 je Zeiteinheit), bei welcher bisher ein zusätzlicher, dem Rotor 6 nachgeschalteter zweiter Aktivator erforderlich war, kann nunmehr die Aktivierungsphase allein auf dem Rotor 6 durchgeführt werden oder der Rotor 6 kann einen deutlich geringeren Durchmesser aufweisen, wenn zwei Aktivationsrotoren vorgesehen werden sollen. Dies bedeutet auch, dass bei reduziertem Maschinenaufwand eine wesentliche Erhöhung der Maschinenleistung möglich ist. In besonderen Fällen kann die Applikationsphase und die Aktivierungsphase auf einem einzigen Rotor durchgeführt werden.

Da das erfindungsgemäße Verfahren auf einer selbsttätigen Regelung zumindest des Volumenstromes v₃ des zu kühlenden Aktivatorgases mittels direkter oder indirekter Kühlung im zweiten Verfahrensschritt der Aktivierungsphase beruht, sind für den Betreiber einer Anlage zeitaufwendige Einstellungen oder Versuche zur Erzielung einer optimalen Sterilisation von Flaschen oder dergleichen Behälter nicht erforderlich. Die jeweilige Anlage kann vielmehr anhand von Herstellerangaben, die verschiedene Behälter-Formen und/oder -Materialien berücksichtigen, problemlos eingestellt und betrieben werden, wobei dann die Aktivierungsphase bzw. die dortigen Verfahrensschritte automatisch durch die anlageninterne Regelung durchgeführt bzw. geregelt werden.

In einer nicht dargestellten Variante der erfindungsgemäßen Sterilisierungsvorrichtung sind Rotor 6 (Aktivatorrotor) Kühlmanschetten vorgesehen sind, welche den zu behandelnden Behälter mindestens teilweise und mindestens zeitweise während der Aktivierung umschließen. Die Kühlmanschette liegt dabei nicht oder nur in Teilflächen an der Behälteroberfläche an, so dass zwischen Behälter und Manschette im bestimmungsgemäßen Betrieb ein Ringspalt oder Kanäle gebildet werden, durch die ein Gas oder eine Flüssigkeit strömen kann. Dabei sind die Manschetten idealerweise so konstruiert, dass diese mindestens eine Öffnung aufweist, welche mit einem Leitungsweg und einer Gasfördervorrichtung verbunden ist, über welche zur Kühlung der Behälterwand ein gasförmiges und/oder flüssiges Medium in den Ringspalt oder die Kanäle geleitet werden kann. Alternativ kann auch über die Öffnung und geeignete Leitungswege und Vakuumpumpen zur Kühlung der Behälterwand kühlere Raum- oder Umgebungsluft in den Ringspalt oder die Kanäle geleitet und dann abführt werden. Mit einer derartigen Kühlmanschette kann sehr schnell eine Sicherheitskühlung erfolgen oder die Todzeit der Temperaturregelung verkürzt werden.

Die wesentlichen Parameter einer Ausführungsform des erfindungsgemäßen Verfahrens zum Sterilisieren von Flaschen 3 mit einem Volumen von 500ml lassen sich wie folgt zusammenfassen:

### Applikationsphase

| | |
|---|---|
| H₂O₂-Konzentration im H₂O₂-Sterilisationsmedium: | 20% |
| maximale Behältertemperatur T_{BW}: | ca. 35°C - 42°C |
| Temperatur T₁: | ca. 145°C |
| Druck des H₂O₂-Sterilisationsmediums: | ca. 0,7 bar |
| Volumenstrom v₁: | ca. 1,5 l/Flasche |
| Volumenstrom v₁: | ca. 2,7 Nm³/h |

### Aktivierungsphase - Verfahrensschritt 1

| | |
|---|---|
| maximale Behältertemperatur T_{BW}: | ca. 67°C - 68°C |
| Temperatur T₂: | ca. 145°C |
| Volumenstrom V₂: | ca. 10,8 l/Flasche |
| | 9,7 Nm³/h |
| Dampfdruck: | ca. 1,0 bar |
| Luftdruck: | ca. 1,5 bar |

### Aktivierungsphase - Verfahrensschritt 2

| | |
|---|---|
| Behältertemperatur T_{BW}: | ca. 67°C - 68°C |
| Volumenstrom v₃: | ca. 10,8 l/Flasche |
| | ca. 9,7 Nm³/h |
| Temperatur T₂ < T₃ | ca. 100°C |

abhängig von Wandstärke und Material
Beimischung von Sterilluft mit Umgebungstemperatur

Die Behandlungszeiten je Aktivierungsphase sind kleiner als 10 s, wobei die Behandlungszeiten x und y unterschiedlich aber auch gleich sein können. Weiterhin ist es möglich, zwischen der Applikationsphase und der Aktivierungsphase beispielsweise eine Behandlungspause von z.B. ca. 4 bis 5 s vorzusehen, d.h. der erste Verfahrensschritt der Aktivierungsphase wird dann mit einer zeitlichen Verzögerung von beispielsweise ca. 5 sec nach dem Einbringen des H₂O₂-Sterilisationsmediums bzw. nach dem Abschluss der Applikationsphase eingeleitet.

Die Erfindung wurde voranstehend an einem Ausführungsbeispiel beschrieben. Es versteht sich, dass zahlreiche Änderungen sowie Abwandlungen möglich sind, ohne dass dadurch der Erfindung zugrunde liegende Erfindungsgedanke verlassen wird. So wurde voranstehend davon ausgegangen, dass die Behandlungsköpfe 6 Teil einer Behandlungsmaschine oder Vorrichtung umlaufender Bauart sind. Selbstverständlich kann das erfindungsgemäße Verfahren auch auf Anlagen durchgeführt werden, die als Linearmaschinen ausgebildet sind. Weiterhin wurde vorstehend davon ausgegangen, dass das Einbringen des H₂O₂-Sterilisationsmediums und das Einbringen des Aktivierungsmediums jeweils über ein und denselben Behandlungskopf 6 erfolgen. Selbstverständlich können in diesen Verfahrensschritten auch unterschiedliche Behandlungsköpfe zur Anwendungen kommen.

## Patentansprüche

1. Verfahren zur Sterilisation von Flaschen, Dosen oder dergleichen Behältern (3) durch Einbringen eines heißen H₂O₂-Sterilisationsmediums in den jeweiligen Behälter (3) in einer Applikationsphase sowie durch Aktivieren des H₂O₂-Sterilisationsmediums in einer Aktivierungsphase durch Einbringen eines Volumenstroms (v₂, v₃) eines sterilen gas- und/oder dampfförmigen heißen Aktivierungsmediums, vorzugsweise durch Einbringen von heißer steriler Luft in den jeweiligen Behälter (3), wobei die Aktivierungsphase wenigstens einen ersten Aktivierungsschritt und zeitlich folgend einen zweiten, zeitlich letzten Aktivierungsschritt aufweist,
dadurch gekennzeichet,
dass in dem zweiten Aktivierungsschritt die Temperatur (T3) des dem jeweiligen Behälter (3) Zugeführten Aktivierungsmediums kleiner ist als die Temperatur (T2) des Aktivierungsmediums im ersten Aktivierungsschritt und in Abhängigkeit von der Behältertemperatur (T_{BW}) oder der Temperatur der Wandung des Behälters (3) geregelt wird.

2. Verfahren nach Anspruch 1, **dadurch gekennzeichnet, dass** die Temperaturregelung durch indirekte Kühlung des Volumenstromes (v₃) der zweiten Aktivierungsphase erfolgt.

3. Verfahren nach Anspruch 1 oder 2, **dadurch gekennzeichnet, dass** die Temperaturregelung durch direkte Kühlung des Volumenstromes (v₃) der zweiten Aktivierungsphase erfolgt, indem ein geregelter Volumenstrom (v₄) an kühlerem Gas dem Volumenstrom (v₃) beigemischt wird und das Gas insbesondere genommen ist aus der Gruppe: sterile Luft, CO2, N2, Edelgase oder einer Mischung aus diesen.

4. Verfahren nach einem der vorangegangenen Ansprüche, **dadurch gekennzeichnet, dass** die Temperatur des Volumenstroms (v₂, v₃) so geregelt wird, dass die Behältertemperatur (T_{BW}) einer Solltemperatur (Soll-T_{BW}) entspricht.

5. Verfahren nach einem der vorangegangenen Ansprüche, **dadurch gekennzeichnet, dass** die Behältertemperatur (T_{BW}) berührungslos gemessen wird.

6. Verfahren nach Anspruch 5, **dadurch gekennzeichnet, dass** die Behältertemperatur (T_{BW}) mit einem Pyrometer gemessen wird.

7. Verfahren nach einem der vorangegangenen Ansprüche, **dadurch gekennzeichnet, dass** in dem zeitlich ersten Aktivierungsschritt der Volumenstrom (v₂) des dem jeweiligen Behälter (3) zugeführten Aktivierungsmediums in Abhängigkeit von der Behältertemperatur (T_{BW}) geregelt wird.

8. Verfahren nach einem der vorhergehenden Ansprüche, **dadurch gekennzeichnet, dass** in dem zeitlich ersten Aktivierungsschritt bei konstanter Temperatur (T₂) oder bei im Wesentlichen konstanter Temperatur und bei konstantem Volumenstrom (v₂) das Zuführen des Aktivierungsmediums in den jeweiligen Behälter (3) zeitgesteuert erfolgt.

9. Verfahren nach einem der vorhergehenden Ansprüche, **dadurch gekennzeichnet, dass** dem jeweiligen Behälter (3) in der Applikationsphase das heiße Sterilisationsmedium mit konstanter Temperatur (T₁) zeitgesteuert zugeführt wird.

10. Verfahren nach einem der vorhergehenden Ansprüche, **dadurch gekennzeichnet, dass** während der Applikationsphase das heiße Sterilisationsmedium dem jeweiligen Behälter (3) mit konstanter oder im Wesentlichen konstanter Temperatur und über eine konstante oder im Wesentlichen konstante Zeitdauer mit einem Volumenstrom (v₁) zugeführt wird, der unter Berücksichtigung der Behältertemperatur (T_{BW}) derart abgestuft oder gesteuert ist, dass die Behältertemperatur (T_{BW}) deutlich unter der Temperatur (T₁) des heißen H₂O₂-Sterilisationsmadiums bleibt.

11. Verfahren nach einem der vorhergehenden Ansprüche, **dadurch gekennzeichnet, dass** während der Applikationsphase das Zuführen des heiße H₂O₂-Sterilisations mediums mit einer Applikationsdauer von 2,5 bis 4 s erfolgt,

12. Verfahren nach einem der vorhergehenden Ansprüche, **dadurch gekennzeichnet, dass** die Behandlungsdauer eines Aktivierungsschrittes kleiner als 10 sec. ist.

13. Verfahren nach einem der vorhergehenden Ansprüche, **dadurch gekennzeichnet, dass** die Aktivierungsphase auf einem einzigen Aktivator durchgeführt wird.

14. Sterilisierungsvorrichtung für Behältern (3) wie Flaschen, Becher, Dosen und dergleichen zur Durchführung des Verfahrens nach einem der vorstehenden Ansprüche, mit wenigstens einem Aktivatorkopf zum Einleiten des heißen Aktivierungsmediums in die Behälter (3), **dadurch gekennzeichnet, dass** diese mindestens eine Vorrichtung zur berührungslosen Temperaturmessung von Festkörperoberflächen und eine hiermit verbundene computergestützte Steuer- und Regeleinrichtung aufweist, die zur Messwertauswertung sowie zur Temperatur- und/oder Volumenstromregelung des dem jeweiligen Behälter (3) zugeführten Aktivierungsmediums zumindest in dem zweiten, zeitlich letzten Aktivierungsschritt der die wenigsten zwei Aktivierungsschritte aufweisenden Aktivierungsphase in Abhängigkeit von der Behältertemperatur (T_{BW}) oder der Temperatur der Wandung des Behälters (3) ausgebildet ist.

15. Sterilisierungsvorrichtung gemäß Anspruch 14, **dadurch gekennzeichnet, dass** an oder im Nahbereich mindestens einer Teilzahl der Aktivatorköpfe (9) Vorrichtungen zur berührungslosen Temperaturmessung von Festkörperoberflächen angeordnet sind, wobei idealerweise an oder im Nahbereich eines jeden Aktivatorkopfes (9) eine Vorrichtung zur berührungslosen Temperaturmessung von Festkörperoberflächen angeordnet ist.

16. Sterilisierungsvorrichtung gemäß Anspruch 14 oder 15, **dadurch gekennzeichnet, dass** die mindestens eine Vorrichtung zur berührungslosen Temperaturmessung von Festkörperoberflächen ein Pyrometer ist.

17. Sterilisierungsvorrichtung gemäß einem der Ansprüche 14 bis 16, **dadurch gekennzeichnet, dass** zur indirekten Regelung der Temperatur durch Beimischung im Leitungsweg mindestens eines Kühlmediums mindestens eine Drossel, ein Ventil oder dergleichen vorgesehen ist und/oder eine Kühleinheit zur indirekten Kühlung in diesem Leitungsweg angeordnet ist.

18. Sterilisierungsvorrichtung gemäß einem der Ansprüche 14 bis 16, **dadurch gekennzeichnet, dass** in jedem Leitungsweg des Kühlmediums stromaufwärts der jeweiligen Aktivatorköpfe (9) mindestens eine Drossel, ein Ventil oder dergleichen angeordnet ist und/oder mindestens eine Kohleinheiten zur indirekten Kühlung in jedem dieser Leitungswege angeordnet ist.

19. Sterilisierungsvorrichtung gemäß einem der Ansprüche 14 bis 18, **gekennzeichnet, durch** wenigstens ein Rohr (10) zum Zuführen des Aktivierungsmediums, wobei an dem Rotor (10) eine Kühlmanschette vorgesehen ist, welche den zu behandelnden Behälter mindestens teilweise umschließt, und wobei die Kühlmenschette an der Behälteroberfläche nicht oder nur teilweise anliegt, so dass zwischen Behälter und Manschette Im bestimmungsgemäßen Betrieb ein Ringspalt oder Kanäle gebildet werden.

20. Sterilisierungsvorrichtung gemäß Anspruch 19, **dadurch gekennzeichnet, dass** die Manschette mindestens eine Öffnung aufweist, welche mit einem Leitungsweg und einer Gasfördervorrichtung verbunden ist, über welche zur Kühlung der Behälterwand ein gasförmiges und/oder flüssiges Medium in den Ringspalt oder die Kanäle geleitet werden kann.

21. Sterilisierungsvorrichtung gemäß Anspruch 19, **dadurch gekennzeichnet, dass** die Manschette mindestens eine Öffnung aufweist, welche mit einem Leitungsweg und einer Vakuumpumpe verbunden ist, worüber zur Kühlung der Behälterwand Umgebungsluft in den Ringspalt oder die Kanäle geleitet und abführt werden kann.

## Claims

1. Method for the sterilisation of bottles, cans, or similar containers (3) by introducing hot H₂O₂ sterilisation mediums into each container (3) during an application phase and by activating the H₂O₂ sterilisation mediums in an activation phase by introducing a volume flow (V₂, V₃) of a sterile gas and/or vapour activating medium, preferably by introducing hot sterile air into each container (3), wherein the activation phase exhibits at least one first activation step and followed in temporal sequence by a second, last activation step,
**characterised in that**
in the second activation step, the temperature (T3) of the activation medium being introduced into the respective container (3) is lower than the temperature (T2) of the activation medium in the first activation step and is regulated as a function of the container temperature (T_{BW}) or the temperature of the wall of the container (3).

2. Method according to claim 1, **characterised in that** the temperature regulation is effected by indirect cooling of the volume flow (V₃) of the second activation phase.

3. Method according to claim 1 or 2, **characterised in that** the temperature regulation is effected by direct cooling of the volume flow (V₃) of the second activation phase, **in that** a regulated volume flow (V₄) of cooler gas is mixed into the volume flow (V3) and the gas is drawn in particular from the group: sterile air, CO2, N2, noble gases, or a mixture of these.

4. Method according to any one of the preceding claims, **characterised in that** the temperature of the volume flow (V₂, V₃) is regulated in such a way that the container temperature (T_{BW}) corresponds to a reference temperature (Soll-T_{BW}).

5. Method according to any one of the preceding claims, **characterised in that** the container temperature (T_{BW}) is measured by contactless means.

6. Method according to claim 5, **characterised in that** the container temperature (T_{BW}) is measured with a pyrometer.

7. Method according to any one of the preceding claims, **characterised in that**, in the temporally first activation step, the volume flow (V₂) of the activation medium conducted into the respective container (3) is regulated as a function of the container temperature (T_{BW}).

8. Method according to any one of the preceding claims, **characterised in that**, in the temporally first activation phase, with constant temperature (T₂) or essentially constant temperature and constant volume flow (V₂), the introduction of the activation medium into the respective container (3).

9. Method according to any one of the preceding claims, **characterised in that** the hot sterilisation medium is conducted into the respective container (3) in the application phase at a constant temperature (T₁) under time control.

10. Method according to any one of the preceding claims, **characterised in that**, during the application phase, the hot sterilisation medium is conducted to the respective container (3) at constant or essentially constant temperature and over a constant or essentially constant period of time with a volume flow (v₁), taking account in such a way that the container temperature (T_{BW}) is staged or controlled in such a way that the container temperature (T_{BW}) remains perceptibly below the temperature (T₁) of the hot H₂O₂ sterilisation mediums.

11. Method according to any one of the preceding claims, **characterised in that**, during the application phase, the introduction of the hot H₂O₂ sterilisation medium takes place with an application duration of 2.5 to 4 s.

12. Method according to any one of the preceding claims, **characterised in that** the treatment duration of an activation step is less than 10 sec.

13. Method according to according to any one of the preceding claims, **characterised in that** the activation phase is carried out on one single activator,

14. Sterilisation device for containers (3) such as bottles, beakers, cans, and the like, for carrying out the method according to one of the preceding claims, with at least one activator head for introducing the hot activation medium into the containers (3), **characterised in that** this exhibits at least one device for contactless temperature measurement of solid body surfaces and a computer-controlled control and regulation device connected to this, which is designed for the measured value evaluation as well as for the regulation of the temperature and/or volume flow of the activation medium conducted to the respective container (3) at least in the second and temporally last activation step of the activation phase, exhibiting at least two activation steps, as a function of the container temperature (T_{BW}) or the temperature of the wall of the container (3).

15. Sterilisation device according to claim 14, **characterised in that**, arranged on or in the close vicinity of at least a part number of the activator heads (9) are devices for contactless temperature measurement of solid body surfaces, wherein, ideally at or in the close vicinity of each activator head (9), a device is arranged for the contactless temperature measurement of solid body surfaces.

16. Sterilisation device according to claim 14 or 15, **characterised in that** the at least one device for contactless temperature measurement of solid body surfaces is a pyrometer.

17. Sterilisation device according to any one of claims 14 to 16, **characterised in that**, for the indirect regulation of the temperature by mixing in the conduction path of at least one cooling medium, at least one choke, one valve, or the like is provided, and/or a cooling unit for indirect cooling is arranged in this conduction path.

18. Sterilisation device according to any one of claims 14 to 16, **characterised in that**, in each conduction path of the cooling medium upstream of the respective activator heads (9), at least one choke, one valve, or the like is arranged, and/or at least one cooling unit for indirect cooling in each of these conduction paths.

19. Sterilisation device according to any one of claims 14 to 18, **characterised by** at least one pipe (10) for the conducting of the activation medium, wherein a cooling sleeve is provided at the rotor (10), which at least partially surrounds the container which is to be treated, and wherein the cooling sleeve is not, or only partially, in contact with the container surface, such that an annular gap or channels are formed between the container and the sleeve when in specified operation.

20. Sterilisation device according to claim 19, **characterised in that** the sleeve exhibits at least one opening, which is connected to a conduction path and a gas delivery device, by means of which, for the cooling of the container wall, a gaseous and/or fluid medium can be conducted into the annular gap or the channels.

21. Sterilisation device according to claim 19, **characterised in that** the sleeve exhibits an opening which is connected to a conduction path and a vacuum pump, by means of which, for the cooling of the container wall, ambient air can be conducted into and out of the annular gap or the channels.

## Revendications

1. Procédé de stérilisation de bouteilles, de boîtes ou de contenants (3) similaires par introduction d'un milieu de stérilisation de H₂O₂ chaud dans les contenants (3) respectifs dans une phase d'application, et par activation du milieu de stérilisation de H₂O₂ dans une phase d'activation, par introduction d'un courant volumique (V₂, V₃) d'un milieu d'activation chaud stérile sous forme de gaz et/ou de vapeur, de préférence, par introduction d'air chaud stérile dans les contenants (3) respectifs, dans lequel la phase d'activation présente au moins une première étape d'activation et, chronologiquement par la suite, une deuxième et chronologiquement dernière étape d'activation,
**caractérisé en ce que**
dans la deuxième étape d'activation, la température (T3) du milieu d'activation acheminé au contenant (3) respectif est inférieure à la température (T2) du milieu d'activation dans la première étape d'activation et est régulée en fonction de la température du contenant (T_{BW}) ou de la température de la paroi du contenant (3).

2. Procédé selon la revendication 1, **caractérisé en ce que** la régulation de la température s'effectue par refroidissement indirect du courant volumique (V₃) de la deuxième phase d'activation.

3. Procédé selon la revendication 1 ou 2, **caractérisé en ce que** la régulation de la température s'effectue par refroidissement direct du courant volumique (V₃) de la deuxième phase d'activation en mélangeant un courant volumique régulé (V₄) de gaz plus froid au courant volumique (V₃) et le gaz est choisi en particulier dans le groupe comprenant : de l'air stérile, du CO₂, du N₂, des gaz rares ou un mélange de ceux-ci.

4. Procédé selon l'une des revendications précédentes, **caractérisé en ce que** la température du courant volumique (V₂, V₃) est régulée de telle sorte que la température du contenant (T_{BW}) corresponde à une température théorique (T_{BW} théorique).

5. Procédé selon l'une des revendications précédentes, **caractérisé en ce que** la température du contenant (T_{BW}) est mesurée sans contact.

6. Procédé selon la revendication 5, **caractérisé en ce que** la température du contenant (T_{BW}) est mesurée avec un pyromètre.

7. Procédé selon l'une des revendications précédentes, **caractérisé en ce que**, dans la première étape d'activation, chronologiquement, le courant volumique (V₂) du milieu d'activation acheminé au contenant (3) respectif est régulé en fonction de la température du contenant (T_{BW}).

8. Procédé selon l'une des revendications précédentes, **caractérisé en ce que**, dans la première étape d'activation, chronologiquement, l'acheminement du milieu d'activation dans le contenant (3) respectif s'effectue de façon programmée dans le temps, à température constante (T₂) ou à température essentiellement constante et à un courant volumique constant (V₂).

9. Procédé selon l'une des revendications précédentes, **caractérisé en ce que** le milieu de stérilisation chaud à température constante (T₁) est acheminé de façon programmée dans le temps, au contenant (3) respectif dans la phase d'application.

10. Procédé selon l'une des revendications précédentes, **caractérisé en ce que**, pendant la phase d'application, le milieu de stérilisation chaud est acheminé au contenant (3) respectif à température constante ou essentiellement constante et sur une durée constante ou essentiellement constante avec un courant volumique (V₁) qui est échelonné ou commandé en tenant compte de la température du contenant (T_{BW}) de telle sorte que la température du contenant (T_{BW}) reste nettement en dessous de la température (T₁) du milieu de stérilisation de H₂O₂ chaud.

11. Procédé selon l'une des revendications précédentes, **caractérisé en ce que**, pendant la phase d'application, l'acheminement du milieu de stérilisation de H₂O₂ chaud s'effectue avec une durée d'application de 2,5 à 4 sec.

12. Procédé selon l'une des revendications précédentes, **caractérisé en ce que** la durée de traitement d'une étape d'activation est inférieure à 10 sec.

13. Procédé selon l'une des revendications précédentes, **caractérisé en ce que** la phase d'activation est réalisée sur un activateur unique.

14. Dispositif de stérilisation pour contenants (3) tels que des bouteilles, des béchers, des boîtes et similaires, pour la réalisation du procédé selon l'une des revendications précédentes, comportant au moins une tête d'activateur pour l'introduction du milieu d'activation chaud dans les contenants (3), **caractérisé en ce que** celui-ci présente au moins un dispositif de mesure de la température sans contact de surfaces de solides et un système de commande et de régulation assisté par ordinateur, relié à celui-ci, qui est conçu pour l'analyse de la mesure et pour la régulation de la température et/ou du courant volumique du milieu d'activation acheminé au contenant (3) respectif au moins dans la deuxième et chronologiquement dernière étape d'activation de la phase d'activation présentant au moins deux étapes d'activation, en fonction de la température du contenant (T_{BW}) ou de la température de la paroi du contenant (3).

15. Dispositif de stérilisation selon la revendication 14, **caractérisé en ce que**, sur ou à proximité d'au moins une partie des têtes d'activateur (9), sont disposés des dispositifs de mesure de la température sans contact de surfaces de solides, dans lequel est disposé idéalement sur ou à proximité de chaque tête d'activateur (9), un dispositif de mesure de la température sans contact de surfaces de solides.

16. Procédé de stérilisation selon la revendication 14 ou 15, **caractérisé en ce que** l'au moins un dispositif de mesure de la température sans contact de surfaces de solides est un pyromètre.

17. Procédé de stérilisation selon l'une des revendications 14 à 16, **caractérisé en ce que**, pour le réglage indirect de la température par mélange dans la voie d'acheminement d'au moins un milieu de refroidissement est prévu(e) au moins une soupape de réduction, un clapet ou similaire et/ou une unité de refroidissement pour le refroidissement indirect est disposée dans cette voie d'acheminement.

18. Procédé de stérilisation selon l'une des revendications 14 à 16, **caractérisé en ce que**, dans chaque voie d'acheminement du milieu de refroidissement, en amont des têtes d'activateur respectives (9), est disposé(e) au moins une soupape de réduction, un clapet ou similaire et/ou au moins une unité de refroidissement pour le refroidissement indirect est disposée dans chacune de ces voies d'acheminement.

19. Dispositif de stérilisation selon l'une des revendications 14 à 18, **caractérisé par** au moins un tube (10) pour l'acheminement du milieu d'activation, dans lequel est prévue, sur le rotor (10), une manchette de refroidissement qui entoure au moins partiellement le contenant à traiter, et dans lequel la manchette de refroidissement ne repose pas, ou repose seulement en partie, sur la surface du contenant de sorte qu'entre le contenant et la manchette soient formés, à l'état de fonctionnement prévu, une fente annulaire ou des canaux.

20. Dispositif de stérilisation selon la revendication 19, **caractérisé en ce que** la manchette présente au moins une ouverture qui est reliée à une voie d'acheminement et à un dispositif de transport de gaz par lequel, pour le refroidissement de la paroi du contenant, un milieu sous forme gazeuse et/ou liquide peut être conduit dans la fente annulaire ou dans les canaux.

21. Dispositif de stérilisation selon la revendication 19, **caractérisé en ce que** la manchette présente au moins une ouverture qui est reliée à une voie d'acheminement et à une pompe à vide, par laquelle pour le refroidissement de la paroi du contenant de l'air ambiant peut être conduit et évacué dans la fente annulaire ou les canaux.
